# EUROPEAN PATENT APPLICATION

(11) **EP 1 452 597 A1**
(43) Date of publication of application: **01.09.2004**
(21) Application number: 02788712.4
(22) Date of filing: 04.12.2002
(51) Int. Cl.: C12N 15/09, C12P 21/00, C12P 21/08, C07K 1/00

(54) **METHOD OF ACTIVATING PROTEIN**

(30) Priority: 04.12.2001 JP 2001370541
(71) Applicant: Mitsubishi Pharma Corporation, Osaka-shi, Osaka 541-0046 (JP)
(72) Inventor: KOUNO, Takaharu, c/o MITSUBISHI PHARMA CORP., Tokyo 103-8405 (JP); KATSUMURA, Yasuhiko, c/o MITSUBISHI PHARMA CORP., Tokyo 103-8405 (JP)
(74) Representative: TER MEER STEINMEISTER & PARTNER GbR
(86) International application number: PCT/JP2002/012701
(87) International publication number: WO 2003/048357

(57) **Abstract**

A method of producing a protein having free cysteine with the use of a serum-free medium, characterized in that the protein is produced in the activated state; a method of producing a protein by culturing in a serum-free medium in accordance with the above method; a method of activating a protein having free cysteine which has been produced in the inactivated state; and a protein obtained by any of the above methods. This protein shows physiochemical or biological properties comparable to a protein obtained by using a serum medium.

## Description

### Technical Field

The present invention relates to a method of producing a protein having free cysteine by using a serum-free medium.

### Background Art

In the conventional recombinant DNA technique, a serum is indispensable when a protein is produced using animal cells as a host. A serum is however expensive and exhibits lot-to-lot variation, so that a severe lot control must be performed upon using it. A serum is derived from a biological material so that mixing of a pathogenic infectious agent such as unknown virus in the serum cannot be denied. It is however difficult to inactivate or remove such an unknown pathogenic infectious agent. Use of serum-free culture is therefore desired when a protein is produced for pharmaceutical use.

When a serum is not used for incubation of animal cells, however, components necessary for maintaining the function of the cells are not adequately supplied and in most cases, a useful protein reaching a satisfactory level cannot be obtained. Moreover, a protein prepared without a serum may be slightly different from the desired one from physical, biochemical and biological standpoints. It is therefore necessary to pay due consideration upon production of a useful protein by culturing without a serum.

The thiol group of free cysteine in a protein which is usually exposed from the molecular surface is not as stable as the disulfide linkage of cysteine which has formed an intermolecular crosslink so that an exchange reaction occurs under weak reductive conditions.

When the thiol group has been subjected to some undesirable modification, the molecule itself loses its original three-dimensional structure by the addition of a strong reducing agent such as dithiothreitol or 2-mercaptoethanol in order to cause the above-described exchange reaction. It is therefore very difficult to proceed with a reaction of a specific portion with good reproducibility when a strong reducing agent is employed.

When the reaction is effected in the presence of only a weak reducing agent, however, there is a fear of an intermolecular disulfide linkage forming a new crosslink and thereby promoting the polymerization of molecules.

When a high-molecular-weight protein is subjected to weak reduction treatment, the progress of the reaction may be insufficient only by the treatment in a basic buffer solution, because the target thiol group portion is not fully exposed to a reagent owing to the disturbance by the three-dimensional structure of the molecule.

It is therefore the common practice in the related art to add a denature reagent such as high-concentration guanidine hydrochloride or urea to the reaction system to modify the molecule and thereby expose the structure, which does not easily appear from the molecular surface in the ordinarily employed buffer solution, to a reagent.

Such a strong modifier, however, induces a nonspecific and irreversible side reaction in a high-molecular-weight protein, leading to the formation of a decomposition product owing to a change in the molecular structure.

Formation of the polymer or side-reaction product such as decomposition product poses a problem in reproducibility or yield upon production of a desired high-purity protein in a large amount. It becomes a serious obstacle to the use of an activated protein as a medicament.

### Disclosure of the Invention

An object of the present invention is to produce, with the use of a serum-free medium, a protein having a similar level of effects to that of a protein produced with the use of a serum medium.

The present inventors have proceeded with the investigation on the reaction conditions. By analyzing the reaction products in detail and overcoming the above-described problems, they have completed the present invention.

The essence of the present invention is as follows:
(1) a method of producing a protein having free cysteine with the use of a serum-free medium, characterized in that the protein having free cysteine is produced in an activated state;
(2) the above-described method, characterized in that the method is carried out in the presence of a reducing agent having capacity low enough not to reduce a disulfide linkage in the protein molecule upon production of the protein but high enough to reduce the modification of free cysteine;
(3) the above-described method, characterized in that the reducing agent is cysteine;
(4) the above-described method, characterized in that the method is performed in the presence of an antioxidant;
(5) the above-described method, characterized in that the antioxidant has capacity enough to inhibit polymerization of proteins via free cysteine;
(6) the above-described method, characterized in that the antioxidant is ascorbic acid;
(7) the above-descried method, characterized in that the method is carried out in the presence of a substance facilitating reduction of the modification of free cysteine;
(8) the above-described method, characterized in that the substance facilitating the reduction of the modification of free cysteine is sodium chloride or potassium chloride;
(9) the above-described method, characterized in that the protein has, in the molecule thereof, free cysteine which has not formed a disulfide linkage and at the same time, has binding activity derived from the thiol group of cysteine;
(10) the above-described method, characterized in that the protein is a recombinant protein;
(11) any one of the above-described methods, characterized in that the protein is an antibody;
(12) the above-described method, characterized in that the antibody has, in the variable region thereof, free cysteine;
(13) the above-described method, characterized in that the antibody is a human monoclonal antibody;
(14) the above-described method, wherein the human monoclonal antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence.Listing;
(15) the above-described method, characterized in that the human monoclonal antibody has heavy-chain hypervariable regions containing an amino acid sequence of SEQ. ID No. 7 of Sequence Listing and light-chain hypervariable regions containing an amino acid sequence of SEQ. ID No. 8 of Sequence Listing;
(16) the above-described method, characterized in that the antibody is a F(ab')₂ fragment antibody;
(17) a method of producing a protein by culturing in a serum-free medium in accordance with the above-described method;
(18) the above-described method, characterized in that the protein has, in the molecule thereof, free cysteine which has not formed a disulfide linkage and at the same time, has binding activity derived from the thiol group of cysteine;
(19) the above-described method, characterized in that the protein is a recombinant protein;
(20) the above-described method, characterized in that the protein is an antibody;
(21) the above-described method, characterized in that the antibody has, in the variable region thereof, free cysteine;
(22) the above-described method, characterized in that the antibody is a human monoclonal antibody;
(23) the above-described method, characterized in that the antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing;
(24) the above-described method, characterized in that the antibody has heavy-chain hypervariable regions containing an amino acid sequence of SEQ. ID No. 7 of Sequence Listing and light-chain hypervariable regions containing an amino acid sequence of SEQ. ID No. 8 of Sequence Listing;
(25) the above-described method, characterized in that the antibody is a F(ab')₂ fragment antibody;
(26) a method of activating a protein which has been produced in the inactivated state and having free cysteine, characterized in that the method is carried out in the presence of a reducing agent, an antioxidant or a substance facilitating the reduction of the modification of free cysteine;
(27) the above-described method, characterized in that the method is carried out in the presence of a reducing agent having capacity low enough not to reduce a disulfide linkage in the molecule of the protein thus produced but high enough to reduce the modification of free cysteine;
(28) the above-described method, characterized in that the reducing agent is cysteine;
(29) the above-described method, characterized in that the antioxidant has capacity enough to inhibit polymerization of proteins via free cysteine;
(30) the above-described method, characterized in that the antioxidant is ascorbic acid;
(31) the above-described method, wherein the substance facilitating the reduction of the modification of free cysteine is sodium chloride or potassium chloride;
(32) the above-described method, characterized in that the protein has, in the molecule thereof, free cysteine which has not formed a disulfide linkage and at the same time, has binding activity derived from the thiol group of cysteine;
(33) the above-described method, characterized in that the protein is a recombinant protein;
(34) the above-described method, characterized in that the protein is an antibody;
(35) the above-described method, characterized in that the antibody has, in the variable region thereof, free cysteine;
(36) the above-described method, characterized in that the antibody is a human monoclonal antibody;
(37) the above-described method, characterized in that the human monoclonal antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing;
(38) the above-described method, characterized in that the human monoclonal antibody has heavy-chain hypervariable regions containing an amino acid sequence of SEQ. ID No. 7 of Sequence Listing and light-chain hypervariable regions containing.an amino acid sequence of SEQ. ID No. 8 of Sequence Listing;
(39) the above-described method, characterized in that the antibody is a F(ab')₂ antibody;
(40) the above-described method, characterized in that the reducing agent, antioxidant or substance facilitating the reduction of the modification of free cysteine is added when the whole antibody exists after purification of the antibody;
(41) a protein obtained by the above-described method;
(42) the above-described protein, characterized in that the protein exhibits physicochemical or biological properties comparable to those of a protein available with the use of a serum medium;
(43) a pharmaceutical composition containing the above-described protein; and
(44) the above-described pharmaceutical composition, characterized in that the composition is an antitumor agent.

### Brief Description of the Drawings

FIG. 1 is a flow chart of production procedures of a GAH antibody;
FIG. 2 illustrates the results of ion exchange HPLC chromatography of a GAH whole antibody, which has been obtained by culturing in a CD CHO medium, after the addition of cysteine,;
FIG. 3 illustrates the results of ion exchange HPLC chromatography of a GAH whole antibody, which has been obtained by culturing in a CD CHO medium, after the addition of cysteine and ascorbic acid;
FIG. 4 illustrates the results of ion exchange HPLC chromatography of a GAH whole antibody, which has been obtained by culturing in an ExCell325-PF medium, after the addition of guanidine hydrochloride;
FIG. 5 illustrates the results of ion exchange HPLC chromatography of a GAH whole antibody, which has been obtained by culturing in an ExCell325-PF medium, after the addition of sodium chloride;
FIG. 6 illustrates the results of ion exchange HPLC chromatography of a GAH whole antibody, which has been obtained by culturing in an ExCell325-PF medium, after the addition of ascorbic acid and sodium chloride;
FIG. 7 illustrates the results of ion exchange HPLC chromatography of a GAH whole antibody which has been obtained by culturing in a serum medium;
FIG. 8 illustrates the results of ion exchange HPLC chromatography of a GAH whole antibody which has been obtained by culturing in a CD CHO medium;
FIG. 9 illustrates the results of ion exchange HPLC chromatography of a GAH whole antibody, which has been obtained by culturing in a CD CHO medium, after the treatment of the present invention;
FIG. 10 illustrates the results of ion exchange HPLC chromatography of a GAH whole antibody, which has been cultured in an ExCell325-PF medium;
FIG. 11 illustrates the results of ion exchange HPLC chromatography of a GAH whole antibody, which has been cultured in an ExCe11325-PF medium, after treatment of the present invention;
FIG. 12 illustrates the results of ion exchange HPLC chromatography of a GAH antibody F(ab')₂ fragment which has been obtained by culturing in a serum medium;
FIG. 13 illustrates the results of ion exchange HPLC chromatography of a GAH antibody F(ab')₂ fragment which has been obtained by culturing in a CD CHO medium;
FIG. 14 illustrates the results of ion exchange HPLC chromatography of a GAH antibody F(ab')₂ fragment, which has been obtained by culturing in a CD CHO medium, after the treatment of the present invention;
FIG. 15 illustrates the results of ion exchange HPLC chromatography of a GAH antibody F(ab')₂ fragment which has been obtained by culturing in an ExCell325-PF medium;
FIG. 16 illustrates the results of ion exchange HPLC chromatography of a GAH antibody F(ab')₂ fragment, which has been obtained by culturing in an ExCell325-PF medium, after the treatment of the present invention;
FIG. 17 illustrates the results of ion exchange HPLC chromatography of a GAH whole antibody which has been obtained by culturing in an ExCell325-PF medium;
FIG. 18 illustrates the results of ion exchange HPLC chromatography of a GAH whole antibody which has been obtained by culturing in an ExCell325-PF medium added with cysteine (1 mM);
FIG. 19 illustrates the results of ion exchange HPLC chromatography of a GAH whole antibody which has been obtained by culturing in an ExCell325-PF medium added with cysteine (5 mM); and
FIG. 20 illustrates the results of ion exchange HPLC chromatography of a GAH whole antibody which has been obtained by culturing in an ExCell325-PF medium added with cysteine (10 mM).

### Best Mode for Carrying Out the Invention

The present invention will hereinafter be described more specifically.

The present invention relates to a method of producing a protein having free cysteine with the use of a serum-free medium and according to this method, the protein having free cysteine can be produced in the activated state.

Examples of such a method include a method of activating a protein having free cysteine in the presence of (1) a reducing agent, (2) an antioxidant, or (3) a substance facilitating the reduction of the modification of free cysteine.

In such a method, (1), (2) and (3) may be added singly, but addition of any two of them in combination ((1) and (2), (1) and (3) or (2) and (3)) is more preferred. Addition of all of (1), (2) and (3) is most preferred.

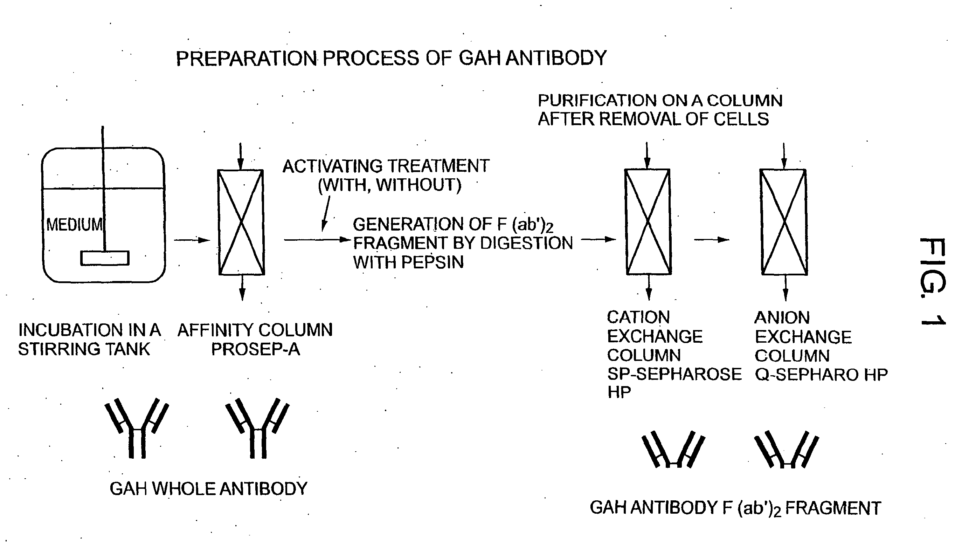

The above-described (1), (2) or (3) may be added at any stage during protein production. In the present invention, however, addition of (1), (2) or (3) upon culturing in a serum-free medium or upon activation of a protein having inactive binding activity into the active one is preferred, with the addition upon activation of a protein having inactive binding activity into the active one being more preferred. For example, when a GAH antibody which will be descried later in Examples is used, any one of the above-described three reagents is added at the time when a whole antibody obtained by purification of the unpurified bulk, which has been produced by culturing in a serum-free medium, exists as illustrated in FIG. 1.

A detailed description will next be made of (1) a reducing agent, (2) an antioxidant, or (3) a substance facilitating the reduction of modification of free cysteine.

As the reducing agent to be used in the present invention, a relatively weak reducing agent is suited, because when the protein is, for example, an antibody, a portion or whole portion of the disulfide linkages in the molecule of the antibody is reduced by a strong reducing agent. Reducing agents having capacity low enough not to reduce the disulfide linkage in the molecule of the protein but high enough to reduce the modification of free cysteine can satisfy the requirements of the reducing agent of the present invention. In the present invention, it is possible to estimate the degree of reducing capacity suited for the present invention, for example, by the method described later in Examples. Although the reducing agent of the present invention is not limited to specific ones, cysteine, for example, is most suited. Addition of from 0.1 to 10 mM, preferably from 0.5 to 5 mM, more preferably from 1 to 3 mM L-cysteine upon conversion into the activated type protein is preferred.

Examples of the antioxidant to be used in the present invention include antioxidants capable of preventing polymerization of proteins via free cysteine. As the antioxidant to be used in the present invention, antioxidants having enough capacity to prevent polymerization of proteins via free cysteine are preferred. The amount of the antioxidant preferable to the present invention can be determined, for example, by the method described later in Examples. Although the antioxidant of the present invention is not limited to specific ones, ascorbic acid, for example, is most preferable. Addition of from 1 to 50 mM, preferably from 5 to 30 mM, more preferably from 10 to 20 mM ascorbic acid upon conversion into the activated type protein can be given as one preferred example.

The term "reducing agent" generally means an agent having action of donating electrons (hydrogen atoms). Antioxidants can be regarded as an agent having reducing action (reducing agent) in the sense that it reduces a disulfide linkage. As described above, however, the reducing agent to be used in the present invention satisfies its requirement when it has reducing capacity low enough not to reduce a disulfide linkage in the molecule of a protein and at the same time, high enough to reduce the modification of free cysteine. In addition, antioxidants having enough capacity to inhibit polymerization of proteins via free cysteine can satisfy the requirement of the present invention.

The present invention is performed in the presence of a substance facilitating the reduction of the modification of free cysteine. As the substance of the present invention facilitating the reduction of the modification of free cysteine, preferred are those capable of enhancing the flexibility of the protein molecule, untangling the protein molecule and exposing free cysteine of the protein (a portion of a thiol group) from the molecular surface thereof within an extent not causing generation of a decomposition product owing to such a change in the molecular structure of the protein.

Although the substance facilitating the reduction of the modification of free cysteine is not limited to a specific one, mono acid-base salts such as sodium chloride and potassium chloride are preferred. More specifically, addition of sodium chloride or the like having a relatively high concentration, for example, from about 0.1 to 4 M, preferably from about 0.25 to 3M, more preferably from about 0.8 to 2M is preferred. Side reactions hardly occur under conditions adjusted as described above. Moreover, the flexibility of the protein molecule is enhanced, and free cysteine to be reduced is likely to be exposed to the reaction so that conversion into the activated type protein is carried out with improved efficiency. As a result, the reaction proceeds with good reproducibility and generation of a decomposition product can be suppressed to a markedly low level. The addition of this substance is therefore particularly helpful for the preparation of a useful protein.

In the present invention, the above-described reagent (1), (2) or (3) may be added to a serum-free culture solution prior to culturing. In the case of the reducing agent (1), similar effects are obtainable by incubation after further addition of L-cysteine to the culture solution to give a final concentration of from 0.1 to 200 mM, preferably from 5 to 100 mM, more preferably from 10 to 50 mM. Adoption of this method to prevent modification of free cysteine with a component in a medium, cause production of an activated type protein directly in the medium and use it immediately after purification is one of useful means for overcoming the problem.

Another subject matter of the present invention resides in a method of activating a protein, which has been produced in the inactivated state and has free cysteine, by treating the protein, after produced in the inactivated state, with the above-described reagent (1), (2) or (3).

The reducing agent, antioxidant and substance facilitating the reduction of the modification of free cysteine which can be employed here are similar to those defined above.

Examples of the medium to be used in the production method and activating method of the present invention include serum-free media not containing a serum such as bovine (fetal) serum in its medium components. Specific examples include serum-free media as described later in Examples such as CD CHO (product of Invitrogen Corporation) and ExCell325-PF (product of JRH Bioscience). In the activating method of the present invention, the medium is not limited to the above-described serum-free medium but a serum medium is usable insofar as it permits production of a protein in the inactivated state. No particular limitation is imposed on the serum concentration of the serum medium insofar as it permits production of a protein in the inactivated state. The concentration is, for example, about 5% or less.

The protein which is a subject of the present invention is characterized by that it has, in the molecular structure thereof, free cysteine. In addition, it is a protein having, in the molecule thereof, free cysteine which has not formed a disulfide linkage and having binding activity derived from the thiol group of cysteine. The term "free (state)" usually means a state in which a substance has an unpaired electron, is relatively unstable, and is abundant in reactivity. In the case of cysteine, its thiol group portion is abundant in reactivity. Free cysteine, particularly, the thiol group portion thereof sometimes takes part in binding activity of a protein. When the protein is an antibody, free cysteine existing in a variable region, particularly, the thiol group portion thereof is presumed to take part in the binding activity with an antigen. More specifically, the protein is preferably an antibody and an antibody having, in the variable region thereof, free cysteine is preferred. A monoclonal antibody is more preferred, with a human monoclonal antibody being most preferred. The human monoclonal antibody is advantageous when it is administered to humans as a medicament, because it is not a protein obtained from different animals.

One of the examples of the human monoclonal antibody which is a subject of the present invention include human monoclonal antibodies having, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing. These amino acid sequences are usually contained in three hypervariable regions of each of heavy chain and light chain in the order of from SEQ. ID Nos. 1, 2 and 3 and SEQ. ID No. 4, 5 and 6, respectively from the N terminal side. The hypervariable region defines the specificity of immunoglobulin as an antibody and binding affinity between an antigen determinant and antigen and it is also called a complementarity determining region. A region other than such a hypervariable region may therefore be derived from another antibody. Human monoclonal antibodies whose amino acids have been modified by substitution, insertion, deletion or addition within an extent not impairing the binding activity (reactivity) with an antigen can also be embraced in the monoclonal antibodies of the present invention.

Specific example includes antibody having a heavy-chain variable region containing an amino acid sequence of SEQ. ID No. 7 of Sequence Listing and a light-chain variable region containing an amino acid sequence of SEQ. ID No. 8 of Sequence Listing, that is, GAH antibody. The GAH antibody is a human monoclonal antibody which has been screened based on the reactivity with gastric cancer and colon cancer and it specifically binds to cancer cells (Japanese Patent Laid-Open No. Hei 4-346918 and Japanese Patent Laid-Open No. Hei 5-304987). This antibody is available by either one of a method of preparing hybridomas between lymphocytes derived from cancer patients and mouse myeloma cells and selecting the hybridoma having the above-described specific amino acid sequence; or a gene engineering technique (Japanese Patent Laid-Open No. Hei 5-304987).

In such a GAH antibody, the 32nd cysteine in SEQ. ID No. 7 of Sequence Listing is characteristic compared with the position of cysteine in the amino acid sequence of a known human antibody. This cysteine is presumed to be free cysteine not taking part in the formation of a disulfide linkage in its molecule. The cysteine residue corresponds to the 4th amino acid in the sequence of SEQ. ID. No. 1 of Sequence Listing and judging from its position in a heavy-chain hypervariable region, it is presumed to participate in the binding activity to an antigen.

As the antibody which is the subject of the present invention, an F(ab')₂ fragment digested from antibody is most preferable, but examples of it include a whole antibody (whole-length antibody, whole portion of the antibody), any antibody fragments (such as F(ab'), F(ab')₂, scFv (single-stranded antibody), etc.) and antibody derivatives.

When the protein, particularly monoclonal antibody, activated by the present invention is used as a medicament, the F(ab')₂ fragmented antibody may be used as well as.the whole antibody.

When the F(ab')₂ fragmented antibody is used as a final product and the method of the present invention is applied thereto in the presence of the reducing agent, antioxidant or the substance facilitating the reduction of the modification of free cysteine, two molecules of F(ab') may presumably be formed because a binding strength at the disulfide linkage portion for crosslinking two F(ab')s is three-dimensionally weak and there exists a molecule which is easily reduced by the treatment under weak reductive conditions of the present invention. In order to avoid formation of such F(ab'), when the F(ab')₂ fragmented antibody is used as a final product, it is effective to carry out the method of the present invention in the stage of a whole antibody after purification of the antibody. The crosslink and three-dimensional structure of the Fc portion are relatively stable in the stage of the whole antibody so that reduction to a monovalent antibody does not occur easily by the method of the present invention. By converting only the thiol group portion taking part in the binding activity into the activated type in the stage of the whole antibody, and gaining the F(ab')₂ fragmented antibody in the activated state by the digestion with pepsin, the problem as described above can be overcome.

The protein available by the method of the present invention has physicochemical or biological properties comparable to those of a protein available by using a serum medium.

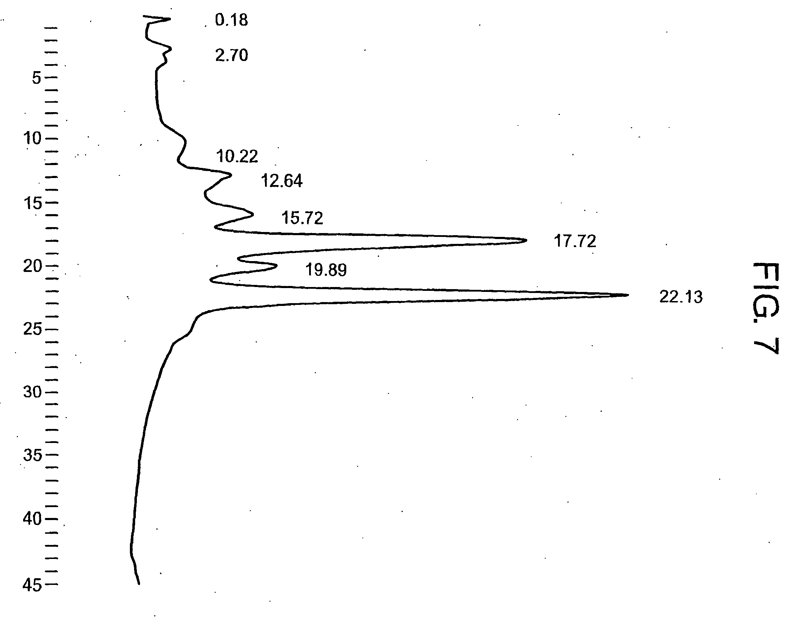

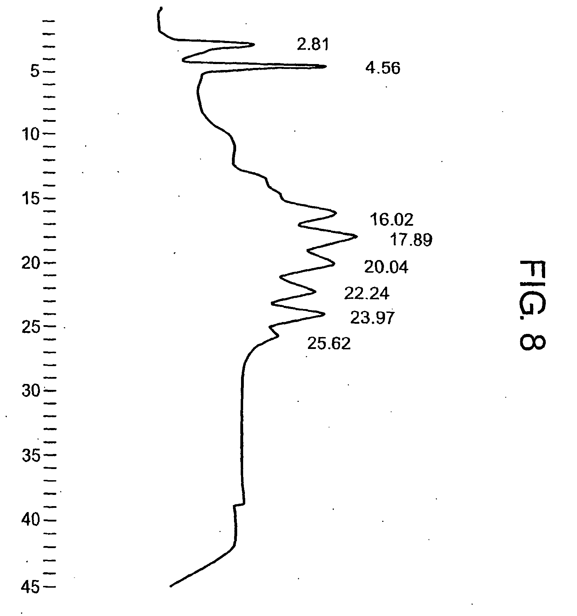

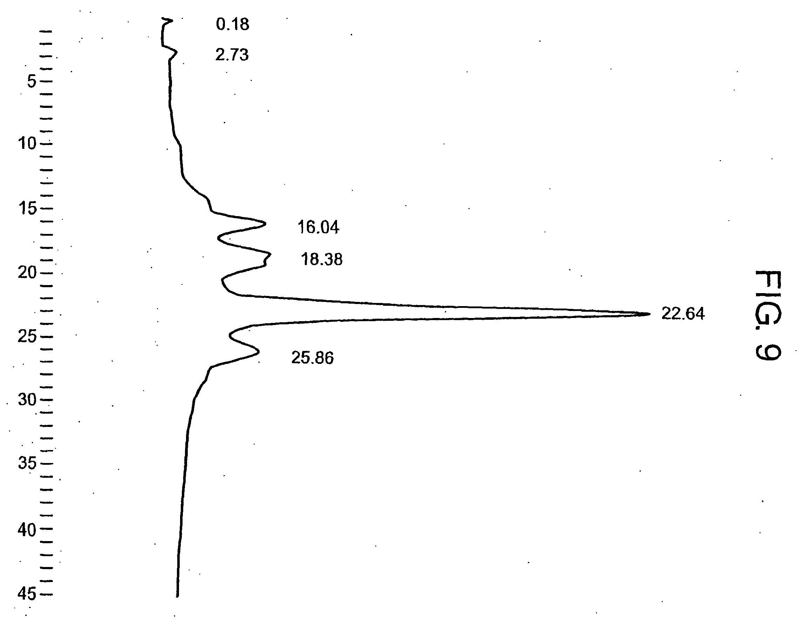

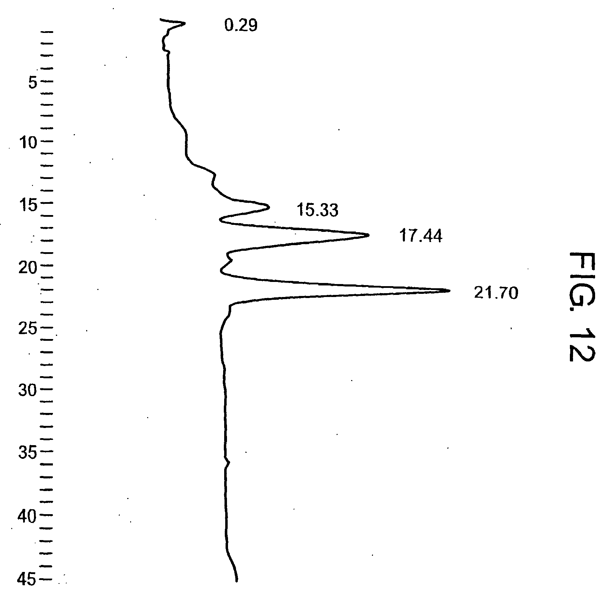

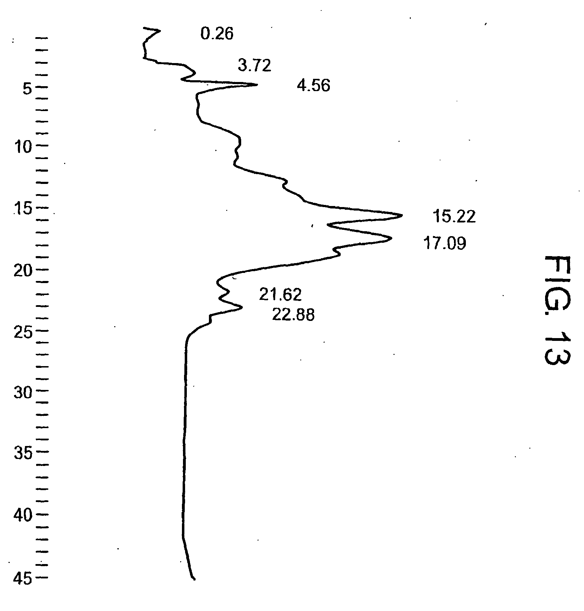

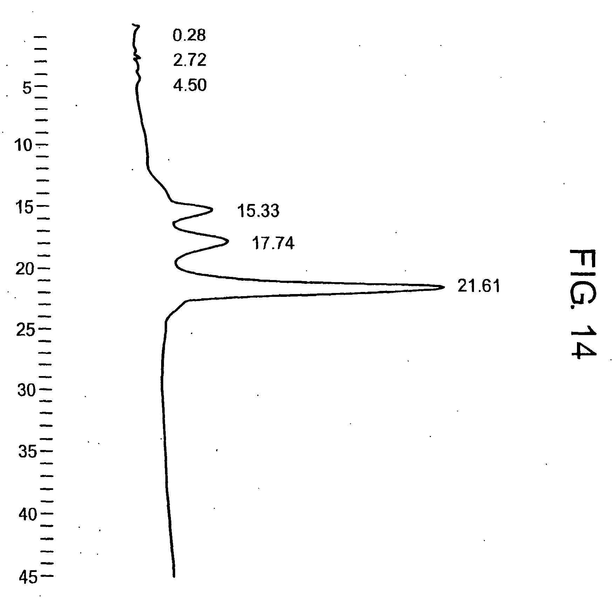

In the present invention, ion exchange high performance liquid chromatography (ion exchange HPLC) is preferred as a method for assaying the physicochemical properties of a protein. Upon ion exchange HPLC, use of conditions which will be described later in Examples is more preferred. The term "comparable" as used herein means that a main peak is observed at a similar retention time to that of the standard product having activity. When the standard product has plural peaks, it means that one or plural peaks are observed at a similar retention time to that of the standard product. In the example which will be described later, a main peak of a GAH antibody is observed at a similar retention time to that of an antibody having activity obtained with the use of a serum medium used as a standard product. More specifically, peaks of the GAH whole antibody are observed at the retention time of about 15.5 minutes, about 17.5 minutes, and about 22 minutes as is apparent from FIG. 7 which will be described later, while those F(ab')₂ fragments of the GAH antibody are observed at the retention time of about 15 minutes, about 17 minutes, and about 21.5 minutes as is apparent from FIG. 12 which will be described later. This retention time is merely an indication and may vary, depending on a difference of analyzers used for measurement or the like. Whether the properties are comparable to those of the standard product or not is estimated based on the comparison of the results of the analysis conducted under the same conditions.

In the present invention, binding activity evaluated by the solid phase enzyme immunoassay (ELISA), particularly under the conditions shown in the below-described examples, is preferably used for assaying biological properties. The term "comparable" used with regards to biological properties means that the activity of the protein has recovered to 80% or greater, preferably 90% or greater, more preferably 100% or greater of that of the standard product.

The present invention provides a medicament containing the protein, which has been obtained by the method of the present invention, as an effective ingredient, for example, a pharmaceutical composition made of the above-described substance and a pharmaceutically acceptable carrier. The present invention provides remedy preparations in various forms. The term "pharmaceutically acceptable carrier" means that the carrier is free from undesirable side effects associated with administration such as nausea, dazzling and vomiting, or free from immunorespohse to the preparation upon frequent administration. An antibody obtained by binding a substance such as toxin to the protein of the present invention can also be used as a pharmaceutical. Examples include a protein-bound, for example, antibody-bound, liposome having a medicament such as antitumor agent, for example, doxorubicin, encapsulated therein (Japanese Patent Laid-Open No. Hei 4-346918, Japanese Patent Laid-Open No. Hei 5-304987 and WO00/64413). The antitumor-substance-containing liposome having an antibody bound thereto can be obtained as a preparation by the known method, for example, a dehydration method (International Patent Publication No. Hei 2-502348), a method of adding a stabilizer and using the mixture in the liquid form (Japanese Patent Laid-Open No. Sho 64-9331) or a lyophilization method (Japanese Patent Laid-Open No. SHo 64-9331). The preparation thus obtained can be administered to patients by intravascular administration, topical administration or the like. The dose can be selected as needed, depending on the kind of an antitumor substance added as an effective ingredient. For example, a liposome having doxorubicin encapsulated therein is administered in an amount of 50 mg/kg or less, preferably 10 mg/kg or less, more preferably 5 mg/kg or less, each in terms of an effective ingredient.

### Examples

The present invention will hereinafter be described in further detail by the following examples. It should however be borne in mind that the present invention is not limited by these examples insofar as it does not depart from the scope of the invention.

### Example 1: Production of the whole antibody of a GAH antibody (which will hereinafter be called "GAH whole antibody") in a medium by serum-free culture

Culture media for cells were prepared by dissolving 4 mmol of glutamine (product of Sigma-Aldrich) and 10 mg of insulin (product of Sigma-Aldrich) in CD CHO (product of Invitrogen Corporation) and ExCell325-PF (product of JRH Bioscience) used serum-free media, respectively, followed by sterile filtration through 0.22 ?m of a bottle top filter (product of Corning Costar). Each of the cell media thus prepared was aseptically charged in a 1L spinner flask (product of Bellco Glass) which had been sterilized in advance in a high-pressure steam sterilizer (product of Sakura Seiki). The flask was then placed in a culture control apparatus (product of Biott) and treated under the following conditions: temperature of 37°C, dissolved oxygen concentration of 3.0 mg/l, pH 7.4 and rotation speed of agitator at 60 rpm.

Recombinant GAH antibody producing CHO cells 1-6R (refer to Japanese Patent Laid-Open No. Hei 5-304987) had been cultured in advance in a roller bottle (product of Falcon). The cells were removed from the roller bottle by adding trypsin, and the cell suspension was collected. After centrifugal separation, the supernatant was discarded. The cells were washed twice with each serum-free medium, whereby seed cells were obtained. After suspension in each serum-free medium and aseptic seeding of the cells in a 1L spinner flask, incubation was started. Sampling was conducted after seeding and a viable cell density and viability were measured by a hemocytometer. As a result, they were 1.09 × 10⁵ cells/ml and 82.3% when cultured in the CD CHO serum-free medium, while they were 0.87 × 10⁵ cells/ml and 84.1% when cultured in the ExCell325-PF serum-free medium.

Incubation was terminated when 353 hours had passed after seeding and the broth was collected. The broth was centrifuged (3000 rpm, 20 min), followed by filtration through a 0.22 ?m bottle top filter, whereby about 800 ml of an unpurified bulk was obtained.

### Example 2: Purification of a GAH whole antibody from the unpurified bulk obtained by serum-free culture

About 800 ml of each unpurified bulk obtained in Example 1 was divided into two portions. They were each purified by column chromatography by using "XK16 column (i.d. 16 mm, product of Amersham Biosciences) filled with Prosep-A resin (product of Millipore) up to a height of 14.3 ml of the column. The unpurified bulk and buffer were fed to the column at a flow rate of 14.3 ml/min with downflow for application and washing and upflow for elution and regeneration. The buffer solutions used for washing, elution and regeneration were 40 mM acetate buffers containing 40 mM NaCl and having a pH of 6.0, 4.0 and 2.7, respectively.

From the unpurified bulks of CD CHO and ExCell325-PF, 47.2 ml and 50.8 ml of whole-antibody-containing solutions (pH 4.0) were obtained. These solutions contain the antibody in an amount of 57 mg and 48 mg, respectively as a result of ultraviolet absorptiometry.

The above-described serum-free culturing steps of the GAH antibody are illustrated in FIG. 1.

### Example 3: Analysis of GAH whole antibody by HPLC

To about 10 ?1 (equivalent to 50 ?g) of the GAH whole antibody produced in the CD CHO medium, a reagent was added so that the composition would be finally a 30 mM tris HCL buffer (pH 9) containing 0.1 mM L-cysteine, whereby the total amount became about 40 ?l. After the mixture was allowed to stand for 16 hours at room temperature, trifluoroacetic acid was added to adjust its pH to 4. The mixture was concentrated by "Microcon YM-10" (product of Amicon) to convert its liquid composition to 0.05% acetic acid. The resulting liquid was subjected to cation exchange liquid chromatography using TSK gel CM-5PW (inner diameter: 7.5 mm, length: 7.5 cm; product of TOSOH). Operation conditions:
Detector: ultraviolet absorptiometer (wavelength measured: 280 nm)
Column: TSKgel CM-5PW (inner diameter: 7.5 mm × length: 7.5 cm) product of TOSOH
Column temperature: a fixed temperature around 30°C
Flow rate: 1.0 ml/min
Mobile phase A: 50 mM sodium phosphate buffer (pH 7.0)
Mobile phase B: 50 mM sodium phosphate buffer (pH 7.0)/0.5M sodium chloride
Feeding of the mobile phase: density gradient control (shown below in Table 1)

**<Table 1>**

| Time after pouring (min) | Mobile phase A (%) | Mobile phase B (%) |
|---|---|---|
| 0 to 40 | 100? 50 | 0 ? 50 |
| 40 to 41 | 50? 100 | 50? 0 |

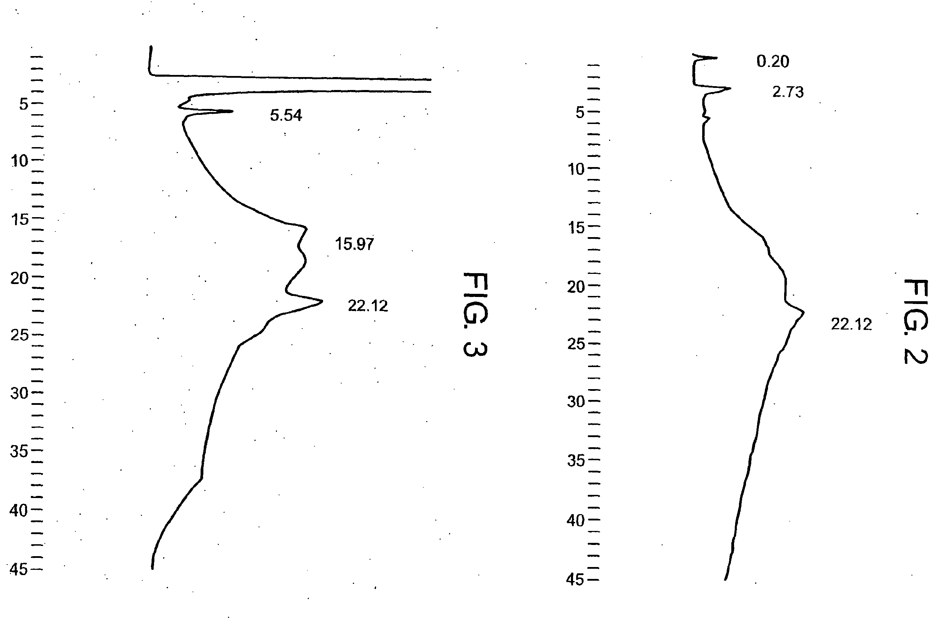

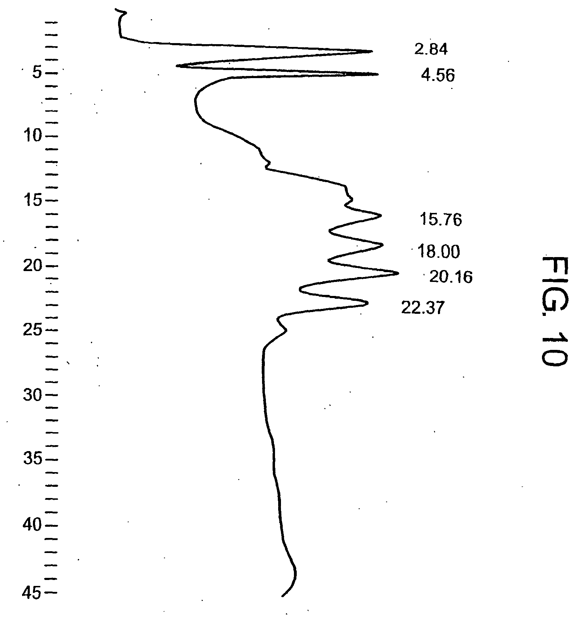

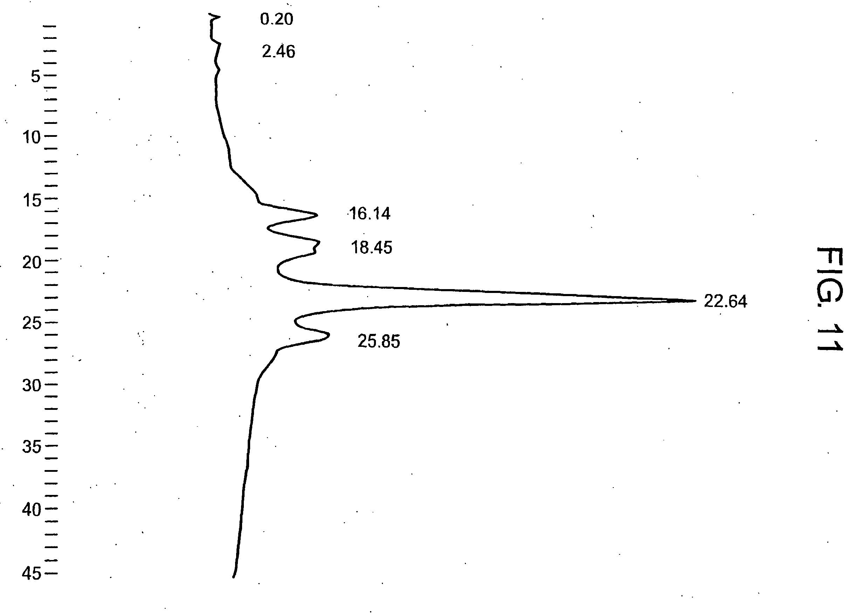

The results are shown in FIG. 2. The chromatogram shows a broad curve with a peak at 22.1 minutes. Compared with FIG. 9, the reaction is presumed to proceed even when a reducing agent (cysteine) is added singly.

### Example 4: Analysis of GAH whole antibody by HPLC

A reagent was added to about 10 ?l (equivalent to 50 ?g) of the GAH whole antibody produced in the CD CHO medium so that the final composition would be composed of a 30 mM tris HCL buffer (pH 9) containing 1 mM L-cysteine and 3 mM ascorbic acid, and the total amount was adjusted to about 80 ?l. After the mixture was allowed to stand for 16 hours at room temperature, trifluoroacetic acid was added to adjust its pH to 4. The mixture was concentrated by "Microcon YM-10" to convert its liquid composition to 0.05% acetic acid. The resulting liquid was subjected to cation exchange liquid chromatography using TSK gel CM-5PW. Operation conditions employed in this example were similar to those employed in Example 3.

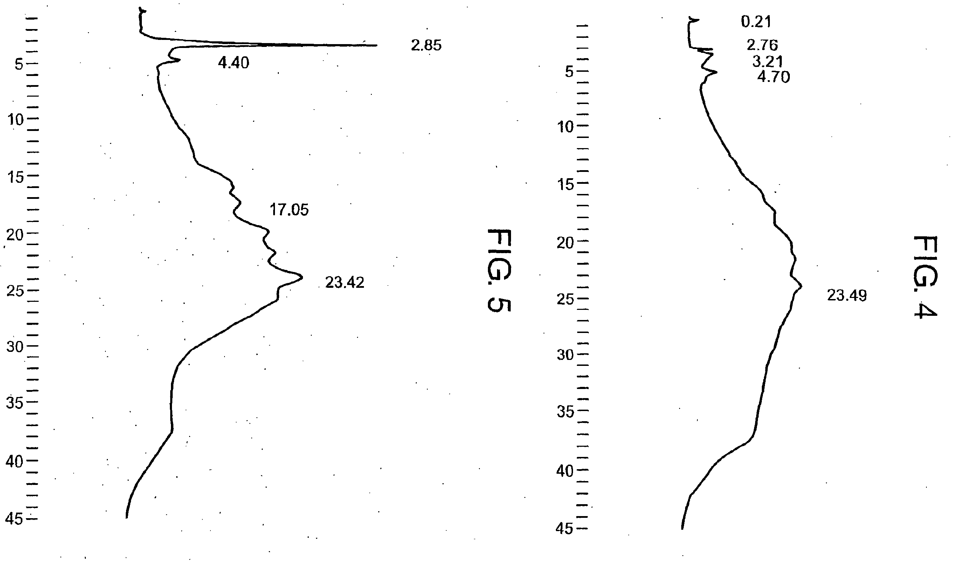

The results are shown in FIG. 3. The chromatogram showed a curve with a peak at 22.1 minutes, but before this curve, it has another broad curve. Compared with FIG. 9, the reaction is presumed to proceed even when only the reducing agent (cysteine) and antioxidant (ascorbic acid) were added.

### Comparative Example 1: Analysis of GAH whole antibody by HPLC

A reagent was added to about 10 ?l (equivalent to 50 ?g) of the GAH whole antibody produced in the ExCell325-PF medium so that the final composition would be composed of a 30 mM tris HCL buffer (pH 9) containing 0.8M guanidine hydrochloride, and the total amount was adjusted to about 80 ?l. After the mixture was allowed to stand for 16 hours at room temperature, trifluoroacetic acid was added to adjust its pH to 4. The mixture was concentrated by "Microcon YM-10" to convert its liquid composition to 0.05% acetic acid. The resulting liquid was subjected to cation exchange liquid chromatography using TSK gel CM-5PW. Operation conditions employed in this example were similar to those employed in Example 3.

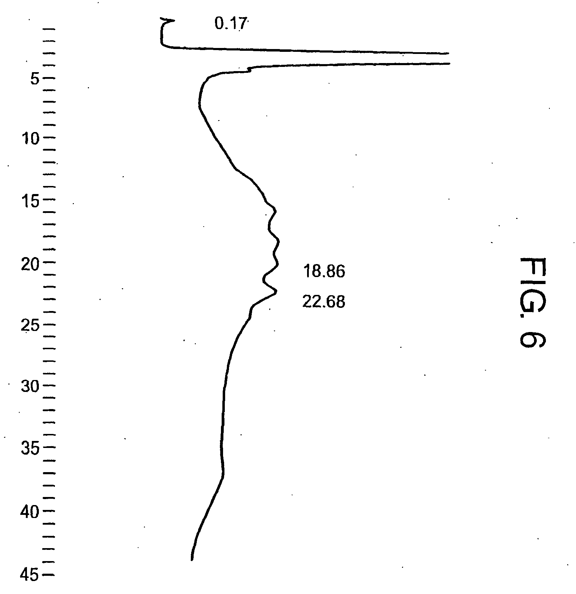

The results are shown in FIG. 4. The chromatogram showed a broad curve without a definite peak, suggesting that the decomposition product was produced owing to the addition of guanidine hydrochloride.

### Example 5: Analysis of GAH whole antibody by HPLC

A reagent was added to about 10 ?l (equivalent to 50 ?g) of the GAH whole antibody produced in the ExCell325-PF medium so that the final composition would be composed of a 30 mM tris HCL buffer (pH 9) containing 2.5M sodium chloride, and the total amount was adjusted to about 90 ?l. After the mixture was allowed to stand for 16 hours at room temperature, trifluoroacetic acid was added to adjust its pH to 4. The mixture was concentrated by "Microcon YM-10" to convert its liquid composition to 0.05% acetic acid. The resulting liquid was subjected to cation exchange liquid chromatography using TSK gel CM-5PW. Operation conditions employed in this example were similar to those employed in Example 3.

The results are shown in FIG. 5. The chromatogram showed a broad curve with a peak at 23.4 minutes. The height of the peak was higher than that of Comparative Example 1 in which guanidine hydrochloride was added singly, suggesting that the production of a decomposition product is rare when sodium chloride is added, compared with that when a strong modifier is added.

### Example 6: Analysis of GAH whole antibody by HPLC

A reagent was added to about 10 ?l (equivalent to 50 ?g) of the GAH whole antibody produced using the ExCell325-PF medium so that the final composition would be composed of a 30 mM tris HCL buffer (pH 9) containing 15 mM ascorbic acid and 3M sodium chloride, and the total amount was adjusted to about 140 ?l. After the mixture was allowed to stand for 16 hours at room temperature, trifluoroacetic acid was added to adjust its pH to 4. The mixture was concentrated by "Microcon YM-10" to convert its liquid composition to 0.05% acetic acid. The resulting liquid was subjected to cation exchange liquid chromatography using TSK gel CM-5PW. Operation conditions employed in this example were similar to those employed in Example 3.

The results are shown in FIG. 6. The chromatogram showed a curve with a peak at 22.7 minutes, but before this curve, it has another broad curve. Compared with FIG. 11 which will be described later, the reaction is presumed to proceed even when only the antioxidant (ascorbic acid) and sodium chloride are added.

### Example 7: Activation treatment of GAH whole antibody

By using "Centricon 30" (product of Amicon), 20 mg of each of the GAH whole antibody solutions obtained in Example 2 with the use of a CD CHO medium and EXCELL325-PF medium, respectively was concentrated to about 1.7 mL. A reagent was added to a 250 ?l portion (equivalent to 3 mg) of the resulting concentrate so that the final composition would be composed of 30 mM tris-HCL buffer (pH 9) containing 1.6M sodium chloride, 2 mM L-cysteine and 12 mM ascorbic acid and the total amount of the mixture was adjusted to about 10 ml. The resulting mixture was allowed to stand for 16 hours at room temperature, followed by the addition of trifluoroacetic acid to pH 4. The mixture was concentrated by "Centricon 30" and the liquid composition was substituted with 0.05% acetic acid.

### Example 8: Analysis of GAH whole antibody by HPLC

The solution obtained in Example 7 was analyzed by cation exchange liquid chromatography and the chromatogram of the solution was compared with that of a sample not subjected to the treatment of Example 7. Operation conditions are similar to those in Example 3. The results are shown in FIGS. 7 to 11.

As a result, it has been recognized that many molecular species derived from heterogeneity exist and retention time is short in the chromatogram of the GAH whole antibody not subjected to the treatment of Example 7 (which may hereinafter be called "invention method") as illustrated in FIG. 8 and FIG. 10. In the chromatogram after the application of the invention method, on the other hand, a main peak is observed at the retention time of 22.6 minutes as can be seen from FIG. 9 and FIG. 11. This peak is presumed to correspond to the peak observed at the retention time of 22.1 minutes of FIG. 7 in which the results of the GAH whole antibody cultured in a serum medium and having binding activity are illustrated. This suggests the smooth progress of conversion into the molecular species in the activated state in the invention method. As illustrated in FIG. 7, several molecular species derived from heterogeneity are recognized also from the activated type GAH whole antibody available by using a serum medium.

### Example 9: Measurement of binding activity of GAH whole antibody

The binding activity of the GAH whole antibodies obtained in Example 2 and Example 7 were analyzed by the ELISA method using an MKN45 cell immobilized plate. Described specifically, MKN45 gastric cancer cells (Naito, et al., Gan to Kagaku Ryoho, 5, 89(1978), obtained from IBL Co., Ltd.) were added to a 96-well plate for cell culture so that each well would contain about 4 × 10⁴ /mL of them, followed by incubation for 2 days. The cells were immobilized by a paraformaldehyde solution and ELIZA plates were prepared. The GAH whole antibody diluted stepwise was added to the plate and it was allowed to stand for 1 hour at 37°C. After washing, horseradish peroxidase labeled goat anti-human IgG antibody was added to each well. After the resulting well was allowed to stand for 1 hour at 37°C, an o-phenylenediamine solution was added for color development. After the reaction was terminated with 2M sulfuric acid, a difference between the absorbance at 490 nm and absorbance at 650 nm was determined by an immunoreader. A calibration curve of the GAH whole antibody cultured using a diluted serum was drawn. Potency was calculated from a ratio of the absorbance of a sample solution having the same concentration to the absorbance based on the calibration curve. The results are shown in Table 2. Table 2 shows the binding activity of the GAH whole antibody and the binding activity of the GAH whole antibody cultured in a serum medium (sample corresponding to that of FIG: 7) is indicated as 100%. The number of the diagram of the ion exchange HPLC of the corresponding sample is shown in the parentheses in the table.

It has been understood that the GAH whole antibody obtained without using the invention method is as low as about 70%, but by the treatment of the invention method, it has increased to 100% or greater, showing good recovery.

**<Table 2>**

| Treatment Medium | Sample not subjected to the treatment of Example 7 | Sample subjected to the treatment of Example 7 |
|---|---|---|
| CD CHO | 70% (sample of FIG. 8) | 117% (Sample of FIG. 9) |
| ExCell325-PF | 69% (sample of FIG. 10) | 131% (Sample of FIG. 11) |

### Example 10: Preparation and purification of F(ab')₂ fragment by pepsin digestion of the whole antibody after activating treatment

A 25 ml portion of the solution obtained in Example 2 was subjected to pepsin digestion without being subjected to activating treatment of Example 7. Described specifically, pepsin (product of Sigma-Aldrich) was added to the solution to give 1.2 mg/g-GAH. After sterile filtration through a Millex filter (product of Millipore, 0.22 ?m), stirring was conducted gently for 17 hours while heating at 37°C.

On the other hand, the activated sample obtained in Example 7 was adjusted pH to 4.0 and then, subjected to pepsin digestion in a manner similar to that described above.

After digestion with pepsin, a GAH antibody F(ab')₂ fragment was purified by cation exchange column chromatography. Described specifically, 15.3 ml of a cation exchange resin "SP-Sepharose FF" (product of Amersham Bioscience) was filled in an KX16 column and the antibody containing solution after pepsin digestion was fed to the column. After washing with a 40 mM acetate buffer solution (pH 4.0) containing 20 mM NaCl, the peak of the antibody was collected while gradually increasing the salt concentration. The flow rate is set at 1.58 ml/min. The volumes of the samples cultured in CD CHO and ExCell325-PF and not subjected to the activating treatment are 12.5 ml and 21.8 ml, respectively, while the volumes of the samples subjected to the activating treatment were 10.5 ml and 11.6 ml, respectively.

The above-described steps for activating treatment after purification of the GAH whole antibody, digestion with pepsin and purification of F(ab')₂ fragment antibody are shown in FIG. 1.

### Example 11: Analysis of GAH antibody F(ab')₂ fragment by HPLC

The GAH antibody F(ab')₂ fragment obtained in Example 10 was concentrated using "Centricon 30", desalted and substituted with a 0.05% acetic acid solution. The resulting solution was subjected to cation exchange liquid chromatography using TSKgel CM-5PW (inner diameter: 7.5 mm, length: 7.5 cm, product of TOSOH). Elution was conducted under similar conditions to those employed in Example 3. The chromatogram of the resulting sample was compared with that of the (Fab')₂ sample prepared while omitting only the treatment of Example 7. Described specifically, each sample was fed to the above-described column equilibrated with a buffer (a 50 mM sodium phosphate buffer (pH 7.0)), followed by elution with a buffer containing 0.5M NaCl. Using, as samples, the GAH antibody F(ab')₂ fragments which had been cultured in a CD CHO medium and an EXCELL325-PF medium and then purified, respectively, those subjected to the treatment of Example 7 and those not subjected to the treatment of Example 7 were compared and the results are shown in FIGS. 12 to 16.

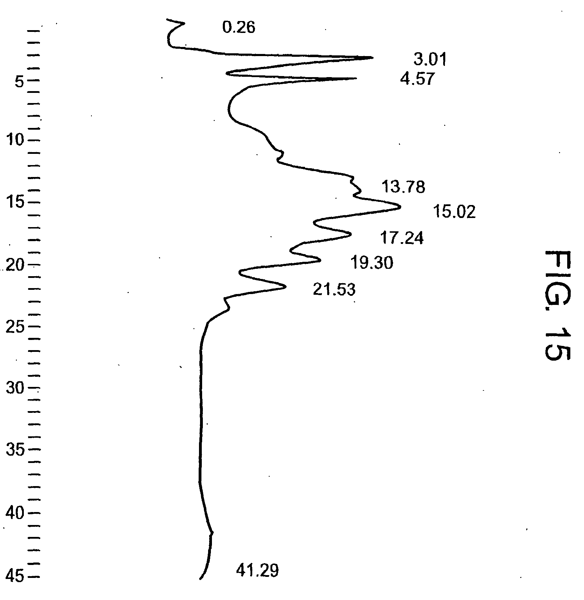

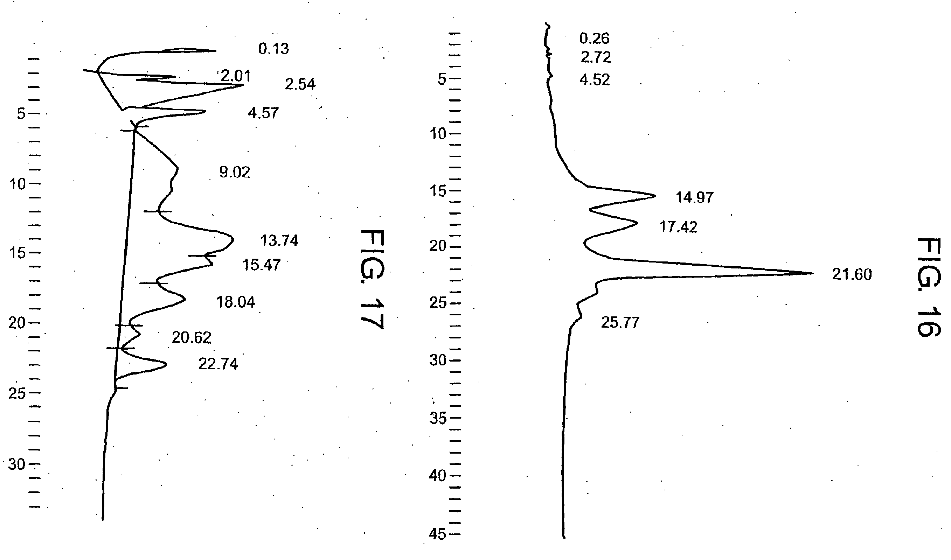

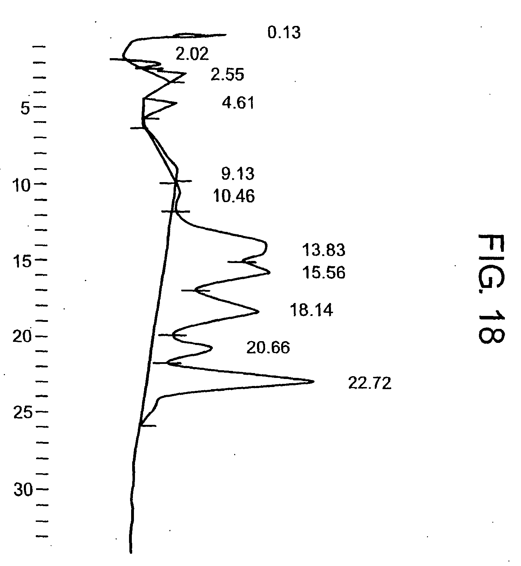

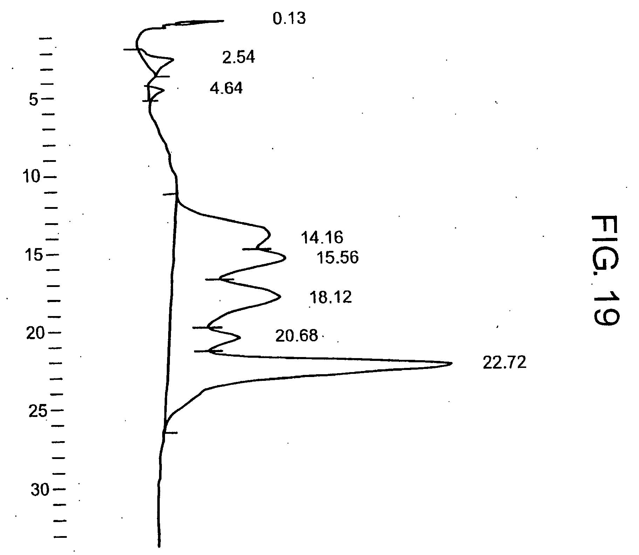

As apparent from FIG. 13 and FIG. 15, a number of molecular species derived from heterogeneity exist in the chromatogram of the antibody F(ab')₂ fragment prepared from the GAH whole antibody obtained without employing the invention method and the retention time is shorter. In the chromatogram of the antibody F(ab')₂ fragment, which has been obtained, in accordance with FIG. 1, from the GAH whole antibody subjected to the invention method, a main peak can be observed at the retention time of 21.6 minutes as illustrated in FIG. 14 and FIG. 16. This peak is presumed to correspond to that of the F(ab')₂ fragment antibody, which has been prepared from the GAH whole antibody having biding activity and cultured in a serum medium, observed at the retention time of 21.7 minutes in FIG. 12. This suggests that the molecular species corresponding to the converted activated type is maintained favorably even in the subsequent steps such as digestion with pepsin and purification on a column. As illustrated in FIG. 12, several molecular species derived from heterogeneity can be observed from the GAH antibody F(ab')₂ fragment in the activated state available by the conventional incubation in a serum medium. Similar patterns are observed in the chromatograms illustrated in FIGS. 14 and 16.

### Example 12: Analysis of binding activity of GAH antibody F(ab')₂ fragment

The binding activity of the GAH antibody F(ab')₂ fragment obtained in Example 10 was analyzed in accordance with the method of Example 9.

The results are shown in Table 3. Table 3 shows the binding activity of the GAH antibody F(ab')₂ fragment and in it, the binding activity of the GAH antibody F(ab')₂ fragment cultured in a serum medium (sample corresponding to that illustrated in FIG. 12) is designated as 100%. The number of the diagram of the ion exchange HPLC to which the sample corresponds to is inserted in parentheses.

The binding activity of the GAH antibody F(ab')₂ fragment available by the pepsin digestion of the GAH whole antibody not subjected to the invention method is as low as about 60%. On the other hand, the GAH antibody F(ab')₂ fragment available by the same treatment of the GAH whole antibody subjected to the invention method is 100% or greater, thus exhibiting good recovery without a deterioration in the binding activity after the steps such as digestion with pepsin and purification by ion exchange column. Its recovery of the binding activity confirmed in the stage of the whole antibody is maintained favorably.

**<Table 3>**

| Treatment Medium | Sample obtained in Example 10 and not subjected to the treatment of Example 7 | Sample obtained in Example 10 and subjected to the treatment of Example 7 |
|---|---|---|
| CD CHO | 70% (sample of FIG. 13) | 105% (Sample of FIG. 14) |
| ExCell325-PF | 60% (sample of FIG. 15) | 110% (Sample of FIG. 16) |

### Example 14: Production of GAH whole antibody in a serum-free medium added with L-cysteine

In 1L of a serum-free medium "ExCell325-PF" (product of JRH Bioscience) were dissolved 4 mmol of glutamine (product of Sigma-Aldrich) and 10 mg of insulin (product of Sigma-Aldrich), followed by sterile filtration through a 0.22 ?m bottle top filter (product of Corning Costar) to prepare a cell culture medium. The medium was collected aseptically in 4 spinner flasks (product of Bellco), each having 250 ml capacity, which had been sterilized in advance. To the flasks, L-cysteine was added aseptically so that it would be 0 mM, 1 mM, 5 mM and 10 mM, respectively. Seedling was conducted into each spinner flask at a density of 8.27 × 10⁵ cells/ml, followed by incubation for 256 hours under the conditions of 37°C and 40 rpm. After centrifugation (3000 rpm, 20 min.), the resulting broth was filtered through a 0.22 ?m bottle top filter, whereby about 140 ml of unpurified bulks were obtained. The unpurified bulks were then subjected to affinity purification in a manner similar to that employed in Example 2, followed by sterile filtration through 0.22 ?m of a bottle top filter. The antibody purified solution thus obtained was analyzed by HPLC as in Example 4.

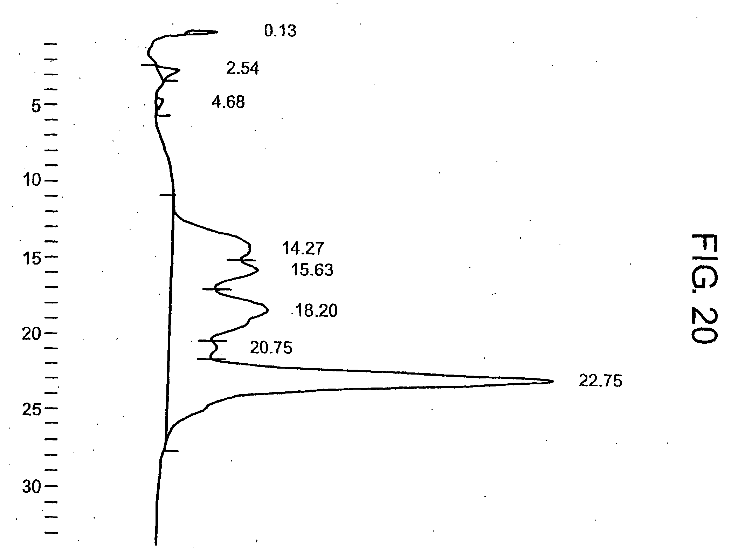

The results are shown in FIGS. 17 to 20. The antibody obtained using a serum-free medium not added with L-cysteine (FIG. 17) and the antibodies to which the invention method had not bee applied after the affinity purification (FIGS. 8 and 10) showed similar chromatographs, suggesting that the their antibody activity is low. On the other hand, the position of the peak is not different between the chromatograph (FIGS. 18 to 20) of the antibodies obtained using a medium added with L-cysteine and the chromatograph (FIG. 7) of the antibody obtained by incubation in a serum medium, suggesting that they have antibody activity. Based on the above-described results, it has been understood that an antibody having a relatively high antibody activity is available by culturing in a serum-free medium added with L-cysteine.

### Industrial Applicability

The method of the present invention makes it possible to produce a protein having free cysteine in the activated state by using a serum-free medium.

The present application is based on and claims priority from Japanese Patent Application No.2001-370541.

## Claims

1. A method of producing a protein having free cysteine with the use of a serum-free medium, **characterized in that** the protein having free cysteine is produced in an activated state.

2. A method according to Claim 1, **characterized in that** the method is carried out in the presence of a reducing agent having capacity low enough not to reduce a disulfide linkage in the protein molecule upon production of the protein but high enough to reduce the modification of free cysteine.

3. A method according to Claim 2, **characterized in that** the reducing agent is cysteine.

4. A method according to any one of Claims 1 to 3, **characterized in that** the method is performed in the presence of an antioxidant.

5. A method according to Claim 4, **characterized in that** the antioxidant has capacity enough to inhibit polymerization of proteins via the free cysteine.

6. A method according to Claim 4 or 5, **characterized in that** the antioxidant is ascorbic acid.

7. A method according to any one of Claims 1 to 6, **characterized in that** the method is carried out in the presence of a substance facilitating the reduction of the modification of free cysteine.

8. A method according to Claim 7, **characterized in that** the substance facilitating the reduction of the modification of free cysteine is sodium chloride or potassium chloride.

9. A method according to any one of Claims 1 to 8, **characterized in that** the protein has, in the molecule thereof, free cysteine which has not formed a disulfide linkage and the protein has binding activity derived from the thiol group of the cysteine.

10. A method according to any one of Claims 1 to 9, **characterized in that** the protein is a recombinant protein.

11. A method according to any one of Claims 1 to 10, **characterized in that** the protein is an antibody.

12. A method according to Claim 11, **characterized in that** the antibody has, in the variable region thereof, free cysteine.

13. A method according to Claim 11 or 12, **characterized in that** the antibody is a human monoclonal antibody.

14. A method according to Claim 13, **characterized in that** the human monoclonal antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing.

15. A method according to Claim 13 or 14, **characterized in that** the human monoclonal antibody has heavy-chain hypervariable regions containing an amino acid sequence of SEQ. ID No. 7 of Sequence Listing and light-chain hypervariable regions containing an amino acid sequence of SEQ. ID No. 8 of Sequence Listing.

16. A method according to any one of Claims 11 to 15, **characterized in that** the antibody is a F(ab')₂ fragment antibody.

17. A method of producing a protein by culturing in a serum-free medium in accordance with a method as claimed in any one of Claims 1 to 16.

18. A method according to Claim 17, **characterized in that** the protein has, in the molecule thereof, free cysteine which has not formed a disulfide linkage and the protein has binding activity derived from a thiol group of cysteine.

19. A method according to Claim 17 or 18, **characterized in that** the protein is a recombinant protein.

20. A methods according to any one of Claims 17 to 19, **characterized in that** the protein is an antibody.

21. A method according to Claim 20, **characterized in that** the antibody has, in the variable region thereof, free cysteine.

22. A method according to Claim 20 or 21, **characterized in that** the antibody is a human monoclonal antibody

23. A method according to any one of Claims 20 to 22, **characterized in that** the antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing.

24. A method according to any one of Claims 20 to 23, **characterized in that** the antibody has heavy-chain hypervariable regions containing an amino acid sequence of SEQ. ID No. 7 of Sequence Listing and light-chain hypervariable regions containing an amino acid sequence of SEQ. ID No. 8 of Sequence Listing.

25. A method according to any one of Claims 20 to 24, **characterized in that** the antibody is a F(ab')₂ fragment antibody.

26. A method of activating a protein which has been produced in an inactivate state and having free cysteine, **characterized in that** the method is carried out in the presence of a reducing agent, an antioxidant and a substance facilitating the reduction of the modification of the free cysteine.

27. A method according to Claim 26, **characterized in that** the method is carried out in the presence of a reducing agent having capacity low enough not to reduce a disulfide linkage in the molecule of the protein thus produced but high enough to reduce the modification of free cysteine.

28. A method according to Claim 26 or 27, **characterized in that** the reducing agent is cysteine.

29. A method according to Claim 26, **characterized in that** the antioxidant has capacity high enough to inhibit polymerization of proteins via the free cysteine.

30. A method according to Claim 26 or 29, **characterized in that** the antioxidant is ascorbic acid.

31. A method according to Claim 26, **characterized in that** the substance facilitating the reduction of the modification of the free cysteine is sodium chloride or potassium chloride.

32. A method according to any one of Claims 26 to 32, **characterized in that** the protein has, in the molecule thereof, free cysteine which has not formed a disulfide linkage and the protein has binding activity derived from a thiol group of free cysteine.

33. A method according to any one of Claims 26 to 33, **characterized in that** the protein is a recombinant protein.

34. A method according to Claim 34, **characterized in that** the protein is an antibody.

35. A method according to Claim 34, **characterized in that** the antibody has, in the variable region thereof, free cysteine.

36. A method according to Claim 34 or 35, **characterized in that** the antibody is a human monoclonal antibody.

37. A method according to Claim 36, **characterized in that** the human monoclonal antibody has, in the heavy-chain hypervariable regions thereof, amino acid sequences of SEQ. ID NOS. 1, 2 and 3 of Sequence Listing and, in the light-chain hypervariable regions, amino acid sequences of SEQ. ID NOS. 4, 5 and 6 of Sequence Listing.

38. A method according to Claim 36 or 37, **characterized in that** the human monoclonal antibody has heavy-chain hypervariable regions containing an amino acid sequence of SEQ. ID No. 7 of Sequence Listing and light-chain hypervariable regions containing an amino acid sequence of SEQ. ID No. 8 of Sequence Listing.

39. A method according to any one of Claims 34 to 38, **characterized in that** the antibody is a F(ab')₂ fragment antibody.

40. A method according to any one of Claims 26 to 39, **characterized in that** the reducing agent, antioxidant or substance facilitating the reduction of the modification of free cysteine is added when a whole antibody after purification exists.

41. A protein obtained by a method as claimed in any one of Claims 1 to 40.

42. A protein according to Claim 41, wherein the protein exhibits physicochemical or biological properties comparable to those of a protein available by using a serum medium.

43. A pharmaceutical composition comprising a protein as claimed in Claim 41 or 42.

44. A pharmaceutical composition, **characterized in that** the composition is an antitumor agent.


SEQUENCE LISTING

<110> MITSUBISHI PHARMA CORPORATION

<120> Methods of activating proteins

<130> 02046W00

<140>
<141>

<150> JP P2001-370541
<151> 2001-12-04

<160> 8

<210> 1
<211> 9
<212> PRT
<213> Homo sapiens

<220>
<223> Inventor: Kouno, Takaharu; Katsumura Yasuhiko

<400> 1
Ile Ser Ser Cys Gly Phe Tyr Trp Asn
 1               5

<210> 2
<211> 12
<212> PRT
<213> Homo sapiens

<400> 2
Ile Gly Tyr Ile Tyr Tyr Ser Gly Ser Thr Tyr Tyr
 1               5                   10

<210> 3
<211> 9
<212> PRT
<213> Homo sapiens

<400> 3
Ser Thr Arg Leu Arg Gly Ala Asp Tyr
1               5

<210> 4
<211> 17
<212> PRT
<213> Homo sapiens

<400> 4
Lys Ser Ser Gln Ser Val Leu Tyr Asn Ser Asn Asn Lys Lys Tyr Leu
1               5                   10                  15
Ala

<210> 5
<211> 7
<212> PRT
<213> Homo sapiens

<400> 5
Trp Ala Ser Thr Arg Glu Ser
1               5

<210> 6
<211> 9
<212> PRT
<213> Homo sapiens

<400> 6

Gln Gln Tyr Tyr Ser Thr Pro Trp Thr
1               5

<210> 7
<211> 119
<212> PRT
<213> Homo sapiens

<400> 7
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Cys
            20                  25                  30
Gly Phe Tyr Trp Asn Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Leu Asp Thr Ser Lys Ser Gln Phe
65                  70                  75                  80
Ser Leu Lys Leu Ser Ser Leu Thr Ala Ala Asp Thr Ala Val Tyr Tyr
                85                  90                  95
Cys Ala Arg Ser Thr Arg Leu Arg Gly Ala Asp Tyr Trp Gly Gln Gly
            100                 105                 110
Thr Met Val Thr Val Ser Ser
        115

<210> 8
<211> 114
<212> PRT
<213> Homo sapiens

<400> 8
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Asn
            20                  25                  30

Ser Asn Asn Lys Lys Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Ser Thr Pro Trp Thr Phe Gly Gln Gly Thr Lys Val Glu Ile
            100                 105                 110
Lys Arg